# EUROPEAN PATENT SPECIFICATION

(11) **EP 1 309 338 B1**
(45) Date of publication and mention of the grant of the patent: **16.11.2005**
(21) Application number: 01956705.6
(22) Date of filing: 17.08.2001
(51) Int. Cl.: A61K 38/17, A61P 35/00

(54) **PHARMACEUTICAL COMPOSITION COMPRISING TRKAIG2 FOR USE IN THE PREVENTION AND/OR TREATMENT OF CANCER**
PHARMAZEUTISCHE ZUSAMMENSETZUNG DIE TRKAIG2 ENTHÄLT ZUR VERWENDUNG IN DER VORBEUGUNG UND/ODER BEHANDLUNG VON KREBS
COMPOSITIONS PHARMACEUTIQUES RENFERMANT UN TRKAIG2, CONVENANT POUR LA PREVENTION ET/OU LE TRAITEMENT DU CANCER

(30) Priority: 18.08.2000 GB 0020504
(43) Date of publication of application: 14.05.2003
(73) Proprietor: The University of Bristol, Clifton, Bristol BS8 1TH (GB)
(72) Inventor: ALLEN, Shelley Jane, Bristol BS1 3NY (GB); DAWBARN, David, Bristol BS1 3NY (GB)
(74) Representative: Dean, John Paul
(86) International application number: PCT/GB2001/003682
(87) International publication number: WO 2002/015924

(56) References cited:
- WO-A-95/25795
- WO-A-96/09387
- WO-A-99/53055
- A. ROBERTSON ET AL.: "Structure of TrkAIg2 and molecular modelling of the neurotrophin binding sites of the Trk receptors." EUROPEAN JOURNAL OF NEUROSCIENCE, vol. 12, no. suppl. 11, 27 June 2000 (2000-06-27), page 329 XP002184446 Oxford, GB
- V. ASOPA ET AL.: "Expression, purification, refolding and characterisation of the immunoglobulin-like sub-domains of the nerve growth factor receptor (TrkA)." BRITISH JOURNAL OF PHARMACOLOGY, vol. 119, no. Proc. Suppl., October 1996 (1996-10), XP001030926 London, GB
- L. O'CONNELL ET AL.: "TrkA amino acids controlling specificity for nerve growth factor." THE JOURNAL OF BIOLOGICAL CHEMISTRY, vol. 275, no. 11, 17 March 2000 (2000-03-17), pages 7870-7877, XP002184445 Baltimore, MD, USA
- P. HOLDEN ET AL.: "Immunoglobulin-like domains define the nerve growth factor binding site of the TrkA receptor." NATURE BIOTECHNOLOGY, vol. 15, no. 7, July 1997 (1997-07), pages 668-672, XP002116514 New York, NY, USA cited in the application

## Description

The present invention relates to the treatment of cancers, in particular, the present invention relates to the treatment of pancreatic cancer.

Nerve growth factor (NGF) and its high-affinity tyrosine kinase receptor A (TrkA) are generally considered to be involved in neural development and survival and growth of central and peripheral nerves.

NGF may be isolated from various sources, most particularly from male mice salivary glands. It may be isolated first as 7S NGF, named for its sedimentation coefficient, which is a complex of β-NGF and γNGF. 2.5S NGF may be obtained from this. 2.5S NGF is known to be responsible for the neurotrophic biological activity of the complex. 2.5S NGF is βNGF but often partially proteolysed at the amino and carboxy termini. NGF is one member of a family of related proteins, the neurotrophins. The other members include for example BDNF, NT-3 and NT-4. All of the neurotrophins bind to a common receptor p75NGFR. Each also binds to one of a homologous family of tyrosine kinase receptors: NGF binds to TrkA, BDNF and NT-4 bind to TrkB, and NT-3 binds to TrkC. NT-3 can also bind TrkA and TrkB with reduced affinity.

Recently two groups have shown that the Ig-like domains of the Trk receptors play important roles in the binding of neurotrophin ligands and receptor activation. Perez P. *et al* (Molecular and Cellular Neuroscience 6: 97-105 (1995)) concluded that both of the Ig-like domains are important for the binding of NGF to TrkA. The co-crystal structure of the NGF homodimer and TrkAIg2 has now been solved (Weismann *et al*. Nature 401, p184-188 (1999)).

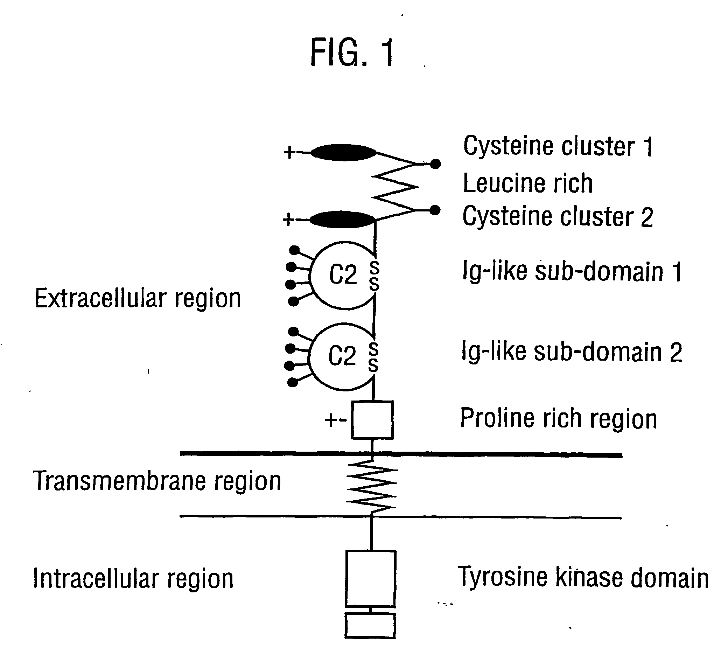

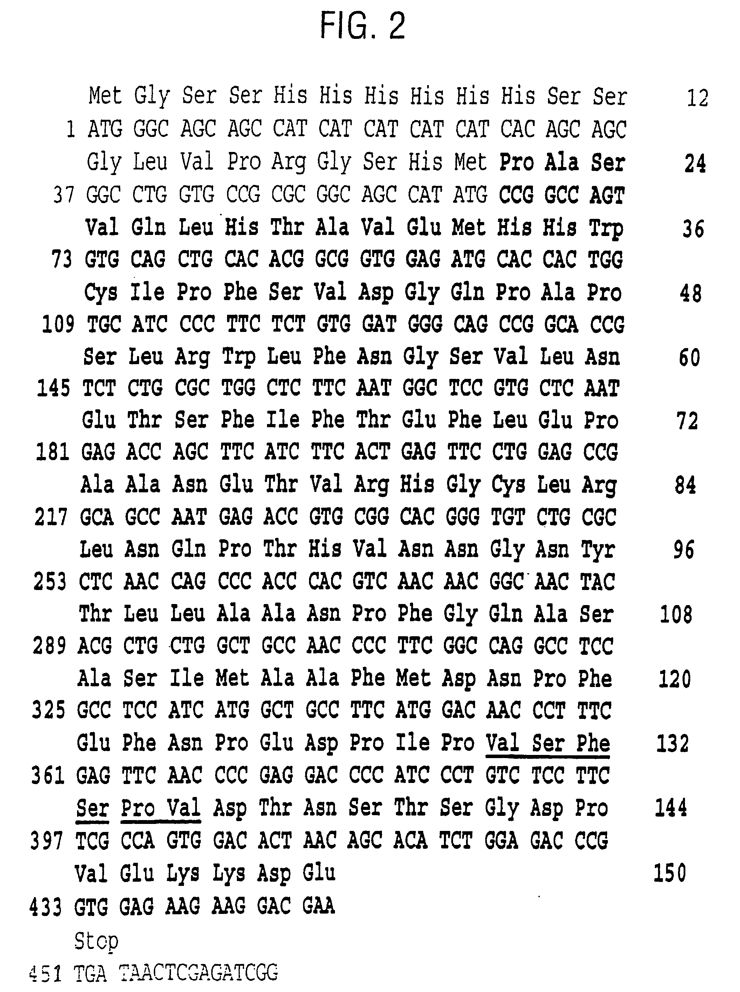

TrkA and isolated domains thereof are further described in WO 99/53055. The accompanying Figure 1 illustrates its structure schematically. The filled circles represent consensus glycosylation sites. TrkAIg2 is defined as including Ig-like sub-domain 2 and the proline rich region. The sequence of TrkAIg2 is shown in Figure 2 which shows the nucleotide sequence and derived amino acid sequence of TrkAIg2 with 6 x His tag. Sequence from human TrkA is in bold, 6 amino acid insert variant is underlined. This sequence includes the human TrkA sequence (amino acids 22 to 150) and a flanking sequence from the pET15b vector (amino acids 1 to 21) which also codes for an N-terminal 6 x His tag. The vector sequence (codons 452 to 468, Figure 2) also provides for a stop codon.

The putative extracellular domain of human TrkA is taken to be either 375 or 381 amino acids long depending on whether the 6 amino acid insert VSFSPV is present. The inventors have recently shown that a protein comprising the two immunoglobulin-like domains and proline-rich region (shown in Fig. 1 as Ig-like subdomain 1, Ig-like subdomain 2 and proline rich region) alone are able to bind NGF with a similar affinity to that of the complete extracellular domain (Holden, P. H. *et al* (1997) Biotechnology 15: 668-672). This region is defined here as TrkAIg1,2. In addition, the inventors have found that an even smaller domain of TrkA referred to as TrkAIg2 (shown in Fig. 2 as amino acids 22 to 150) is able to bind NGF with a similar affinity to the complete extracellular domain or the TrkAIg1,2 region and is thus responsible primarily for its binding properties. TrkAIg2 is defined herein as including the TrkAIg-like sub-domain2 together with the proline rich region, spanning amino acids 22 to 150 as defined in Figure 2 and may also contain amino acids 1 to 21, and may or may not include the six amino acid insert VSFSPV as shown as amino acids 130 to 135 also in Figure 2.

The pancreas is a gland which makes pancreatic enzymes for digestion of food. These are released into ducts which pass into the bile duct and into the duodenum. The pancreas also produces several hormones, including insulin.

Cancer of the pancreas is the fifth highest cause of cancer-related death in the Western world. It accounts for 2% of newly diagnosed cancers in the US each year, but 5% of all cancer deaths, and has the poorest survival rate of all of the major malignancies. Over 26,000 people in the US present with cancer of the pancreas each year. Men have a higher incidence of pancreatic cancer and resulting mortality rate than women. Those of Afro-Caribbean descent have incidence and mortality rates that are about 50% higher than the rates for Caucasians, whilst the rates for Hispanics and the Asian-American groups are generally lower.

Most pancreatic cancers are adenocarcinomas arising from the ducts. The disease is often advanced by the time symptoms present, with less than 5% of sufferers surviving after 5 years, as successful treatment is rare. 2% of pancreatic cancers are islet cell cancer (i.e. cancers of the islets of Langerhans that produce insulin and other hormones). These have a better prognosis. As pancreatic cancer grows, the tumour may invade organs that surround the pancreas, such as the stomach or small intestine. Pancreatic cancer cells may also metastasise and spread to other parts of the body, often forming new tumours in lymph nodes, the liver, and sometimes in the lungs or bones.

When symptoms appear, they depend on the location and size of the tumour. For example, if the tumour blocks the common bile duct so that bile cannot pass into the intestines, the skin and whites of the eyes may become yellow, and the urine may become dark, i.e. jaundice.

As the cancer grows and spreads, pain often develops in the upper abdomen and sometimes spreads to the back. The pain may become worse after the person eats or lies down. Cancer of the pancreas can also cause nausea, loss of appetite, weight loss and weakness.

Islet cell cancer can cause the pancreas to make too much insulin or other hormones. When this happens, the person may feel weak or dizzy and may have chills, muscle spasms, or diarrhoea.

The progression of the pancreas is difficult to control. This disease can currently be cured only if diagnosed at an early stage. Cancer that begins in the pancreatic ducts may be treated with surgery, radiation therapy, or chemotherapy or a combination. Islet cell cancer is usually treated with surgery or chemotherapy. A total pancreatectomy, removing the entire pancreas as well as the duodenum, common bile duct, gallbladder, spleen, and nearby lymph nodes, may be necessary.

Pain is a common problem, only partially alleviated by pain killers, or other treatments, such as injecting alcohol into the area around nerves to block the pain, or cutting the nerves in the abdomen during surgery. Cancer of the pancreas and its treatment may interfere with production of pancreatic enzymes and insulin. As a result, patients may have problems digesting food and maintaining the proper blood sugar level.

The reasons for the high frequency of perineural invasion and the presence of the pain in pancreatic cancer are not clear. NGF is involved in stimulating epithelial cancer cell growth and perineural invasion as well as in pain generation in chronic benign disorders. NGF and TrkA have been examined by Northern blot analysis, *in situ* hybridisation and immunocytochemistry in normal and pancreatic tissue samples (Zhu, ZW, *et al* (1999) Journal Of Clinical Oncology Vol.17, No.8, pp.2419-2428). Northern blot analysis showed that NGF and TrkA mRNA levels were significantly increased in pancreatic cancer tissues. *In situ* hybridisation and immunocytochemistry showed a strong presence of NGF in the cytoplasm of pancreatic cancer cells and TrkA was intensely present in the perineurium of pancreatic nerves. It has also been shown that levels of endogenous NGF in pancreatic cancer correlates with degree of perineural invasion and pain. Thus enhanced expression of the NGF/TrkA system may influence perineural invasion (Zhu, ZW, *et al* (1999) Journal Of Clinical Oncology Vol.17, No.8, pp.2419-2428).

International patent application WO 99/11291 discloses a method of treating human brain tumor cells comprising transfecting the cells with a gene encoding the full TrkA receptor. NGF is added and leads to the death of the transfected cells. This disclosure is clearly different from the present invention because cells are transfected with a gene encoding the full TrkA receptor and because it is necessary to add NGF.

The inventors have unexpectedly shown that the growth rate of at least two pancreatic cancer cell lines is inhibited by the presence of TrkAIg2 and at certain higher concentrations cell death is induced.

The inventors have unexpectedly discovered that TrkAIg2 is capable of inhibition of cancer cell growth and mediates cell death.

Accordingly, a first aspect of the present invention provides the use of TrkAIg2, or a functional portion or derivative capable of preventing the growth of cancer cells, in the preparation of a medicament for the treatment and/or prevention of a cancer in a patient.

The medicament may be in a form for ingestion, intravenous injection, intradermal, intraperitoneal, intracerebroventricular or direct application to the tumour site.

The cancer may be pancreatic cancer, or may be selected from other cancers, such as, breast cancer, prostate cancer, brain tumours such as glioblastoma, neuroblastoma, skin cancer and lung cancer. Preferably the cancer is pancreatic cancer.

A second aspect of the invention provides a method of inhibiting tumour cell growth *in vitro*, the method comprising contacting cells with TrkAIg2 or an analogue thereof.

The term "TrkAIg2" as used herein means the Ig-like sub-domain 2, preferably with the proline rich sequence, which is shown as amino acids 22 to 150 in Figure 2. Preferably TrkAIg2 also includes a 6 x His tag. It is particularly preferred that TrkAIg2 includes the flanking sequence from vector pET15b, which comprises a 6 x His tag, and is shown as amino acids 1 to 21 in Figure 2.

The term "analogue" used in relation to TrkAIg2 refers to functional portions and derivatives of the natural TrkAIg2 sequence. The functional portions and derivatives must retain the function of the full TrkAIg2 sequence, i.e. they must be capable of preventing the growth of cancer cells. Methods for testing the function of portions and derivatives of TrkAIg2 are described in the examples below. An examples of a derivative of TrkAIg2 is the splice variant of TrkAIg2, which does not have the the 6 amino acid insert underlined in Figure 2 (amino acids 130 to 135). The splice variant of TrkAIg2 (i.e. without the 6 amino acid insert) is normally associated with neurons rather than mast or non-neuronal cells. Derivatives of TrkAIg2 includes sequences from other biological sources such as mammals, birds (for example chicken), insects, reptiles or amphibian. Derivatives include variants of the foregoing sequences as a result of the degeneracy of the genetic code and insertion, deletion and substitution variants. Preferably the derivatives have a homology of at least 80%, more preferably at least 90% and most preferably at least 95% to the TrkAIg2 sequence shown in Figure 2. Homology is preferably determined using BLAST. Preferably the derivatives differ by only 1 to 10 amino acids from the sequence of TrkAIg2 given in Figure 2. It is further preferred that any amino acid changes are conservative. Conservative changes are those that replace one amino acid with one from a family of amino acids which are related in their side chains. For example, it is reasonable to expect that an isolated replacement of a leucine with an isoleucine or valine, an aspartate with a glutamate, a threonine with a serine, or a similar conservative replacement of an amino acid with a structurally related amino acid will not have a major effect on the biological activity of the protein. Mutations which increase the number of amino acids which are capable of forming disulphide bonds with other amino acids in the protein can also be made in order to increase the stability of the protein. Other mutations which increase the desired function of the protein can also be made.

Pharmaceutical compositions of this invention comprise TrkAIg2 or an analogue thereof, with any pharmaceutically acceptable carrier, adjuvant or vehicle. Pharmaceutically acceptable carriers, adjuvants and vehicles that may be used in the pharmaceutical compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene- polyoxypropylene-block polymers, polyethylene glycol and wool fat.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant such as Ph. Helv or a similar alcohol.

Embodiments of the invention will now be described, by way of example only, and with reference to the accompanying figures in which:
Figure 1 shows schematically the structure of TrkA;
Figure 2 show the nucleotide and amino acid sequence of TrkAIg2 with a 6 His tag.
Figure 3 is a graph showing the reduction in cell metabolic rate with increasing concentrations of TrkAIg2 in the Mia Pa Ca2 pancreatic cancer cell line;
Figure 4 is a photomicrograph of Mia Pa Ca2 pancreatic cancer cell line without (a) and with (b) TrkAIg2 showing dramatic cell death;
Figure 5 shows the effect of addition of TrkAIg2 to human pancreatic cell line PANC-1 on cell viability after [A] 24 hours [B] 48 hours [C] 72 hours [D] 96 hours incubation. The negative control is taken as the metabolic activity of cells at time 0 hours;
Figure 6 is a photomicrograph of Mia Pa Ca2 cells stained with an antibody to TrkAIg2 [mnuA2]; and
Figure 7 is a photomicrograph of staining with antibody to p75 receptor [p75NGFR Me20.4].[a] A875 cells which express large quantities of p75NGFR [b] Mia Pa Ca2 cells.

### EXAMPLE 1

### Inhibitory action of TrkAIg2 on pancreatic cancer cells

### 1. Pancreatic cancer cell line MIA-Pa-Ca-2 (ECACC No. 85062806)

TrkAIg2 was prepared as described in WO 99/53055.

Human pancreatic cancer cell line MIA-Pa-Ca-2 ECACC No. 85062806 (European Collection, Porton Down)

The cells were established from tumour tissue of the pancreas of a 65 year old male Caucasian. The cells can be cloned in soft agar and are sensitive to asparaginase, and when taken at passage number 135 have epithelial morphology.

Cells were taken from liquid nitrogen, thawed at 37°C , and maintained in culture for 3 weeks. MIA-Pa-Ca-2 cells were detached and resuspended in 2x DMEM, 20% FCS, penicillin/streptomycin and 100µl plated out at a density of 4 x 10³ cells/well in a 96 well plate. Serial dilutions (1:2) of TrkAIg2 were made in sodium phosphate 20mM, sodium chloride 100mM, pH7.4 and an equal volume was immediately added at a range of final concentration of 3.25uM to 0.01uM. A 'buffer only' control was included. After 48 hours without refeeding cell metabolic activity was determined using Promega's CellTitre 96® cell proliferation assay.

The CellTiter 96® Assay is a non-radioactive, colorimetric assay for measuring metabolic activity of viable cells. The assay is composed of solutions of (3-(4,5-dimethylthiazol-2-yl) -5-(3-carboxymethoxyphenyl)-2-(4-sulfophenyl)-2H-tetrazolium, inner salt; MTS) and an electron coupling reagent (phenazine methosulfate; PMS). MTS (Owen's reagent) is bioreduced by cells into a formazan that is soluble in tissue culture medium. The conversion of MTS into the aqueous soluble formazan is accomplished by dehydrogenase enzymes found in metabolically active cells. The quantity of formazan product as measured by the amount of 490nm absorbance is proportional to the number of living cells.

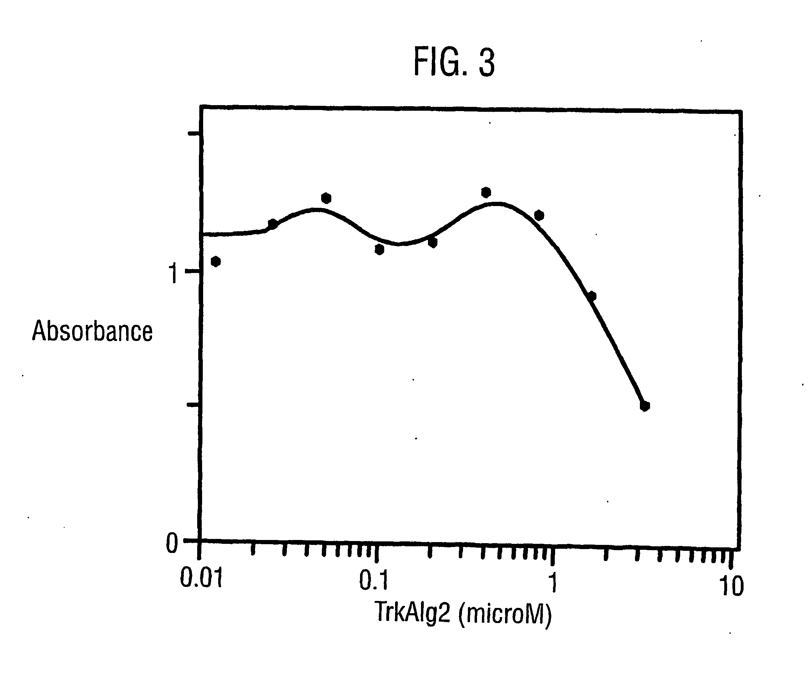

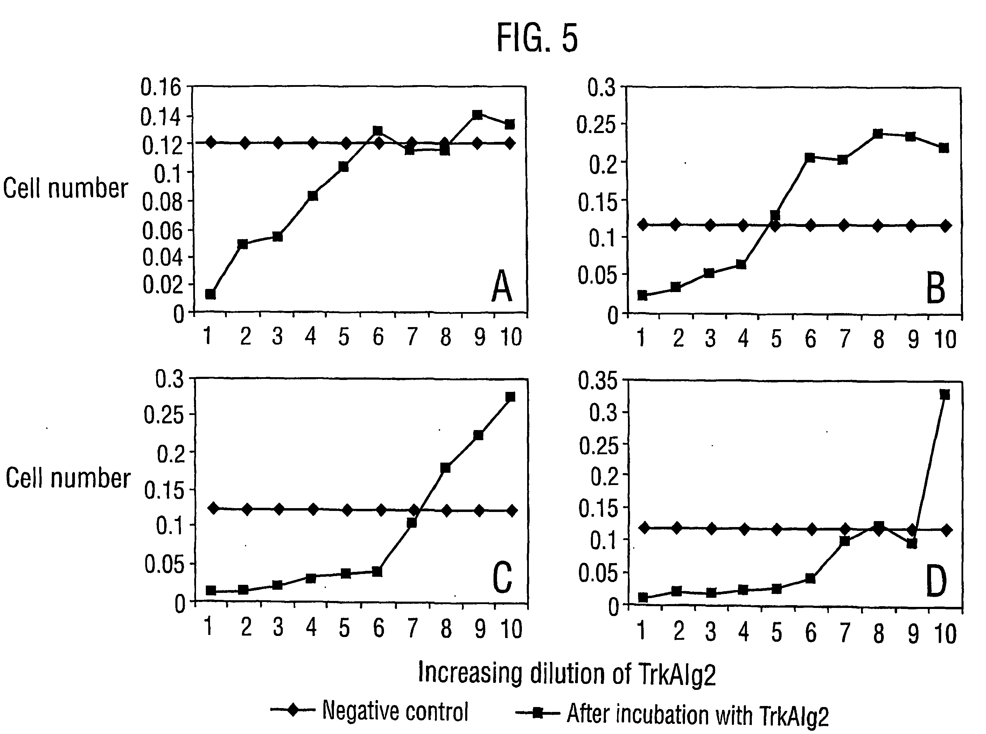

The results are shown in Fig. 3 which is a graph of the observed reduction in metabolic rate with increasing concentrations of TrkAIg2 (µM). Duplicate wells were visualized using an inverted phase microscope. Fig 4A shows Mia Pa Ca2 cells without the presence of Ig2.

### 2. Pancreatic cancer cell line PANC1 ECACC No. 87092802

The cells were established from ductal tumour tissue of the pancreas of a 56 year old male Caucasian.

Cells were taken from liquid nitrogen, thawed at 37°C, and maintained in culture for 3 weeks. MIA-PA-CA-2 cells were detached and resuspended in 2x DMEM, 20% FCS, penicillin/streptomycin and 100µl plated out at a density of 5 x 10³ cells/well in a 96 well plate. Serial dilutions (1:2) of TrkAIg2 were made in sodium phosphate 20mM, sodium chloride 100mM, pH7.4 and an equal volume was diluted in 2xDMEM serum free medium 1:1 added at a range of final concentration of 4.7µM to 0.02µM to start concentration in 1xDMEM medium.

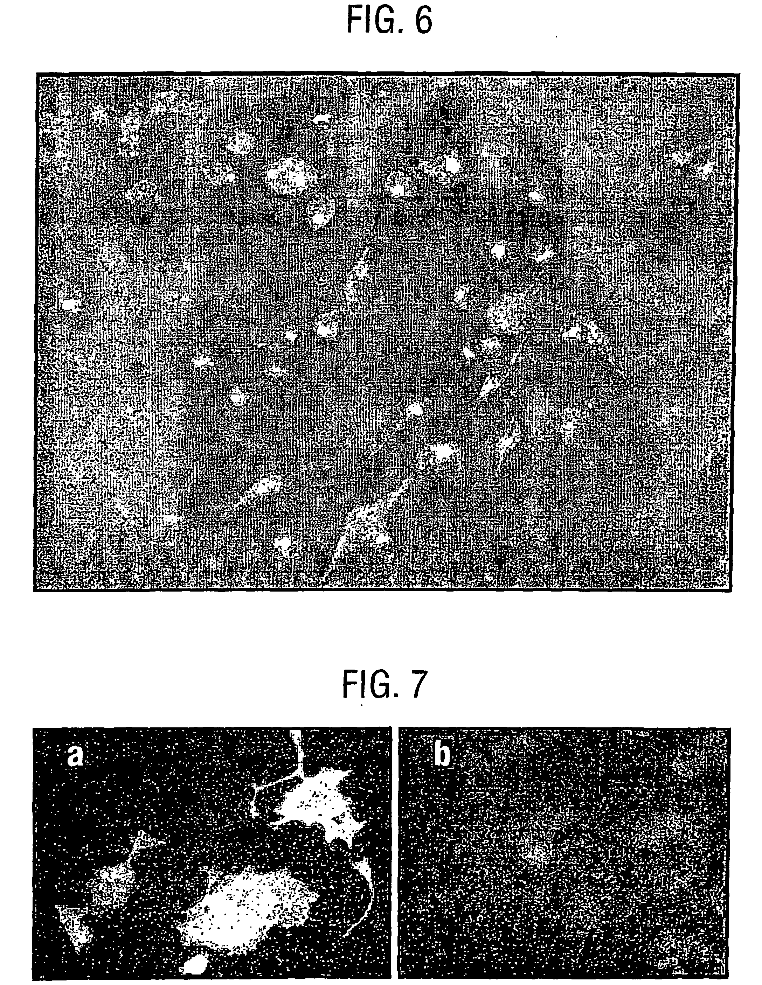

Mia Pa Ca2 cells were grown on Essex-Henley slides and incubated with antibodies to TrkAIg2 (mnuA2) or p75 (HB8737 me20.4) followed by anti-rabbit IgG-FITC conjugated or anti-mouse FITC conjugated respectively. Cells were visualized using a Leitz microscope with fluorescence module. Cells were shown to express TrkA receptors (Fig. 6) but not p75 (Fig.7b). By contrast, a positive control, A875 cells which express p75NGFR receptors did stain with this antibody (Fig. 7a).

The results are shown in Fig. 5 from 4.7 to 0.03uM TrkAIg2 caused cell death and from 0.30 to 0.02µM, it inhibited cell growth.

Studies suggest that other types of cancer may also rely on the presence of NGF for growth and proliferation (Sortino, M. A. *et al* (2000) *Molecular Endocrinology* Vol. 14 (No. 1): 124-136; Walch, E. T. *et al* (1999) *Clinical & Experimental Metastasis Vol. 17 (No.4)*: 307-314; Descamps, S. *et al* (1998) *Journal of Biological Chemistry* Vol. 273 (No. 27): 16659-16662)

### Prostate Cancer

NGF may play a role in some prostate cancers. Studies on the androgen-dependent, prostate adenocarcinoma LNCaP cell line (Sortino, M. A. *et al* (2000) *Molecular Endocrinology* Vol. 14 (No. 1): 124-136) show that application of NGF results in a concentration-dependent increase in proliferation. This is accompanied by an enhanced expression of prostate-specific antigen (PSA) and added to the proliferative effect of dihydrotestosterone. The proliferative effect of NGF appeared to be mediated by TrkA. TrkA but not p75(NGFR) was expressed in LNCaP cells; but both the proliferative response and the phosphorylation of TrkA upon NGF treatment were prevented by the tyrosine kinase inhibitor K252a. LNCaP cells transiently transfected with the cDNA encoding for p75(NGFR) appeared more sensitive to NGF, and increased in number when exposed for 72 h to NGF compared with wild LNCaP cultures.
Furthermore, Walch *et al*., using this same human prostate cancer cell line (LNCaP), and also PC-3, and DU145, demonstrate that NGF and NT-4 increase *in vitro* invasion (Walch, E. T. *et al* (1999) *Clinical & Experimental Metastasis **Vol. 17** (No.4)*: 307-314). In addition the expression of heparanase, a molecular determinant of tumour metastasis, was found to be induced. The effects were most marked in the DU145 cells. It is reported that these lines had negligible TrkA and TrkC expression, although TrkB was expressed in all three prostatic tumour cell lines examined. The DU145 cells were also positive for p75(NGFR). The study showed that NGF and NT4 are important in metastasis and that their expression coincides with transformation to a malignant phenotype capable of invasion along the perineural space and extracapsular metastasis to distant sites.

These facts make it highly likely that certain prostate tumours may respond well to treatment with TrkAIg2 which will sequester endogenous NGF.

### Breast Cancer

There also seems to be good evidence to support a role of NGF in breast cancer. Descamps and colleagues (Descamps, S. *et al* (1998) *Journal of Biological Chemistry* Vol. 273 (No. 27): 16659-16662) show that NGF is able to stimulate the proliferation of breast cancer cells (MCF-7 and MDA-MB-231 cell lines), although it is unable to stimulate growth of normal breast epithelial cells. This abnormal stimulation induces cells in the G(0) phase to re-enter the cell cycle, as well as shortening cell cycle duration. The two cancer cell lines and the normal breast cell line express TrkA and p75(NGFR) receptors. Activation of mitogen-activated protein kinase can be detected in breast cancer cells after 10 min of NGF stimulation, whereas no change was detected in normal breast cells.

Of course this may not be true of all breast cancer cell lines. However, Tagliabue *et al.* show TrkA mRNA in 12 of 14 human breast carcinoma specimens and three of four cell lines (Tagliabue, E. *et al* (2000) *Journal of Biological Chemistry* Vol. 275 (No. 8): *5388-5394)*. NGF stimulated two of the three TrkA-expressing cell lines. Importantly, inhibition of NGF-induced activation by an antibody directed against the extracellular domain of TrkA (but not by an inhibitor of only TrkA phosphorylation) demonstrated the requirement of NGF binding but not of TrkA kinase activity of MAPK activation, suggesting that recruitment of another kinase for transmission of the mitogenic signalling. This means that in order to stop the NGF-induced stimulation in these cells, it is necessary to remove or inhibit the effect of NGF on the extracellular region of the TrkA receptor.

It seems likely therefore that treatment with TrkAIg2 will be of benefit to patients with breast cancer.

### Brain Tumour

Metastatic tumour cells in the brain which attach to endothelial cells and respond to brain-derived invasion factors, can invade the blood-brain barrier. In responsive tumour cells, neurotrophins promote invasion by enhancing the production of basement-membrane-degradative enzymes, such as gelatinase and heparanase, which cause a local breakdown of the blood-brain barrier. Menter and colleagues (Menter D. G. *et al* (1994) *Involvement of Neurotrophins and Growth-Factors in Brain Metastasis Formation Invasion & Metastasis Vol. 14* (No. 1-6): 372-384) found increased levels of NGF in tumour-adjacent tissues at the invasion front of human melanoma tumours in the brain. In addition, the proliferation of a glioblastoma cell line (87 HG 31) could be stimulated by NGF (Delman N *et al* (1995) Cancer Research Vol. 55 (No. 10): 2212-2219). The addition of TrkAIg2 to these tissues is expected to result in a decrease in tumour proliferation.

### Lung Cancer

Clonal growth of three lung cancer cell lines (HTB 119, HTB 120, CCL 185) could be stimulated up to 3-fold by NGF with a dose-response relationship (0.5-500 ng/ml) (Oelmann, E. *et al* (1995) *Cancer Research* Vol. 55 (**No.10**): 2212-2219). This effect was completely reversible by anti-NGF antibody and by the tyrosine kinase inhibitor genistein.

### Epithelial Cancer

NGF has been suggested to be a potent regulator of cell proliferation in human epithelial cells (Di Marco, *et al* (1993). *Journal Of Biological Chemistry*, 268, 22838- 22846). Normal human keratinocytes synthesize and secrete biologically active NGF in a growth regulated fashion (DiMarco E., *et al* (1991) *Journal Of Biological Chemistry* 266, 21718-21722). Keratinocytes express both the low (p75(NGFR)- and the high-affinity (TrkA) NGF- receptors. NGF upregulates the expression of NGF mRNA (Pincelli, C. and Marconi, A. (2000) *Journal of Dermatological Science **Vol*****.** ***22 (No. 2):** 71-79).* K252, which inhibits trk phosphorylation, blocks NGF-induced keratinocyte proliferation and induces apoptosis in normal keratinocytes. Furthermore, normal keratinocytes over-expressing either TrkA or NGF proliferate better than controls (Pincelli, C. and Marconi, A. (2000) *supra)*.

Therefore, in view of the previous NGF studies carried out by the inventors, it is possible that NGF is involved in cell proliferation, particularly with reference to tumourous cells. It seems likely that in many of these cell types the addition of the NGF sequestering agent TrkAIg2 will inhibit proliferation and may, as in pancreatic tumour cell lines, cause actual cell death on application. However, this theory has never previously been expressed or tested.

## Claims

1. Use of TrkAIg2, or a functional portion or derivative capable of preventing the growth of cancer cells, in the preparation of a medicament for the treatment and/or prevention of cancer in a patient

2. A use according to claim 1, wherein the medicament is in a form for ingestion, intravenous injection, intradermal, intraperitoneal, intracerebroventricular or direct application to the tumour site.

3. A method of inhibiting tumour cell growth *in vitro*, the method comprising contacting cells with TrkAIg2, or a functional portion or derivative capable of preventing the growth of cancer cells.

4. A use according to claim 1 or 2 or a method according to claim 3 wherein the cancer is a pancreatic cancer.

5. A use according to claim 1 or 2 or a method according to claim 3 wherein the cancer is breast cancer, prostate cancer, brain tumours, skin cancer or lung cancer.

## Patentansprüche

1. Verwendung von TrkAIg2, oder einem funktionalen Teil oder Derivat, der bzw. das in der Lage ist, das Wachstum von Krebszellen zu verhindern, bei der Herstellung eines Medikaments zur Behandlung und/oder Prävention von Krebs bei einem Patienten.

2. Verwendung nach Anspruch 1, worin das Medikament in einer Formulierung zur Einnahme, intravenösen Injektion, intradermalen, intraperitonealen, intrazerebroventrikulären oder direkten Anwendung an der Tumorstelle vorliegt.

3. Verfahren zur Hemmung des Wachstums von Tumorzellen *in vitro,* wobei das Verfahren den Kontakt der Zellen mit TrkAIg2 oder einem funktionalen Teil oder Derivat, der bzw. das in der Lage ist, das Wachstum von Krebszellen zu verhindern, umfasst.

4. Verwendung nach Anspruch 1 oder 2 oder ein Verfahren nach Anspruch 3, worin der Krebs Pankreaskrebs ist.

5. Verwendung nach Anspruch 1 oder 2 oder ein Verfahren nach Anspruch 3, worin der Krebs Brustkrebs, Prostatakrebs, Gehirntumore, Hautkrebs oder Lungenkrebs ist.

## Revendications

1. Utilisation de TrkAIg2, ou d'une partie fonctionnelle ou d'un dérivé fonctionnel capable d'empêcher la croissance des cellules cancéreuses, dans la préparation d'un médicament destiné au traitement et/ou à la prévention du cancer chez un patient.

2. Utilisation selon la revendication 1, dans laquelle le médicament est sous une forme destinée à une ingestion, à une injection intraveineuse, intradermique, intrapéritonéale, intracérébroventriculaire ou à une application directe au site tumoral.

3. Procédé d'inhibition de la croissance des cellules tumorales *in vitro,* le procédé comprenant la mise en contact des cellules avec TrkAIg2, ou une partie fonctionnelle ou un dérivé fonctionnel capable d'empêcher la croissance des cellules cancéreuses.

4. Utilisation selon la revendication 1 ou 2, ou un procédé selon la revendication 3, dans lequel le cancer est le cancer du pancréas.

5. Utilisation selon la revendication 1 ou 2, ou un procédé selon la revendication 3, dans lequel le cancer est le cancer du sein, le cancer de la prostate, les tumeurs cérébrales, le cancer de la peau ou le cancer des poumons.
